# EUROPEAN PATENT SPECIFICATION

(11) **EP 2 406 809 B1**
(45) Date of publication and mention of the grant of the patent: **15.10.2014**
(21) Application number: 10713359.7
(22) Date of filing: 12.03.2010
(51) Int. Cl.: H01J 35/08, A61B 6/00

(54) **X-RAY SCANNERS AND X-RAY SOURCES THEREFOR**
RÖNTGENSCANNER UND RÖNTGENQUELLEN DAFÜR
SCANNERS À RAYONS X ET SOURCES DE RAYONS X POUR CES APPAREILS

(30) Priority: 12.03.2009 GB 0904236
(43) Date of publication of application: 18.01.2012
(73) Proprietor: CXR Limited, Radlett Hertfordshire WD7 8HT (GB)
(72) Inventor: MORTON, Edward, James, Guildford Surrey GU1 2SL (GB)
(74) Representative: Wilson, Alan Stuart
(86) International application number: PCT/GB2010/050438
(87) International publication number: WO 2010/103331

(56) References cited:
- WO-A1-2004/091405
- DE-A1- 4 410 757
- US-A- 5 570 403
- US-A- 5 651 047
- US-A1- 2001 050 972
- US-A1- 2010 020 934

## Description

### Field of the Invention

The present invention relates to X-ray scanners and in particular to scanners arranged to use different energies of X-rays for use, for example, in the imaging or analysis of objects.

### Background

A new generation of multi-focus X-ray tubes are now being designed to address problems in imaging systems which involve rapid movement of the object under inspection. This is particularly important in tomographic imaging systems where object motion can create unacceptably high levels of artefact in reconstructed images. To address this problem, multi-focus X-ray sources are proposed in which often many hundreds of individual electron guns are arranged, typically into a circular array, and each electron gun is switched on sequentially to irradiate a respective point on a circular anode with the same radius as that of the electron guns. This forms a rotating X-ray source without the need for physical motion of the assembly, hence creating the opportunity for very high speed tomographic imaging.

In such tomographic X-ray systems, it is often desirable to provide materials discrimination capability which is typically achieved through the use of the reconstructed grey level of the tomographic image with calibration back to a set of known reference standards (e.g. air, water, aluminium).

It is recognised that further materials discrimination capability can be achieved when the energy spectrum of the X-ray beam is taken into account since each spectral component in the incident X-ray beam is attenuated to a different amount by each component material within the object under inspection. Low atomic number materials provide modest attenuation of low energy X-rays whilst high atomic number materials provide significant attenuation of low energy X-rays. By analysis of the X-ray spectrum after filtering by the object, it is possible to obtain further materials discrimination than if the X-ray spectrum is simply integrated.

In a practical X-ray system, it is expensive to measure the energy of every single X-ray photon that arrives at the detector. This is because the arrival rate of photons at each detector element A, is relatively high (often over 1M Hz photon arrival rate) and the complexity and associated power dissipation of the detection electronics becomes a significant issue.

One means to simplify the situation is to utilise more than one inexpensive integrating detector per imaging channel, but with a filter placed between one detector and the other. The filtered detector is generally made thick to measure the high energy components of the X-ray beam transmitted through the object. The unfiltered detector is usually quite thin and so responds preferentially to the low energy components of the transmitted X-ray beam.
US570403 discloses a multiple energy scanning system with a plurality of detecting systems of different energy characteristics and an x-ray source switching between energies. WO2004/091405 discloses a dual energy scanning system using dual energy detectors, exposing the object to multiple energies simultaneously, wherein the multiple energies are provided by a filter having sections with different filter characteristics.

### Summary of the Invention

The present invention provides an X-ray scanner comprising an electron source and an anode, the anode having a target surface formed from a plurality of different materials. The electron source is arranged to direct a beam of electrons at the target surface so as to generate, simultaneously, X-rays of two different energy spectra from the two materials. The scanner further comprises two detector arrays having different response characteristics. For example one array may be more sensitive to X rays having one of the energy spectra, and the other array may be more sensitive to X-rays having the other of the energy spectra.

The detectors of one of the arrays of detectors may include a filter material and a detector element. The two materials may each be arranged to generate X-rays having intensity peak at a respective fluorescent energy. The filter material may be arranged to provide different attenuations at the two fluorescent energies. The filter material may have an absorption edge at a frequency between the two fluorescent energies.

The target surface may have a plurality of material areas each formed from one of the materials, and the electron source may be arranged to direct a beam of electrons at a target area of the target, the target area comprising part of at least two of the material areas.

The electron source may be arranged to direct electrons at a plurality of target areas spaced along the target. The material areas may be arranged as parallel strips each extending through a plurality of the target areas. The target surface may be formed from a mixture of the materials.

Preferred embodiments of the present invention will now be described by way of example only with reference to the accompanying drawings.

### Brief Description of the Drawings

**Figure 1** is schematic view of a known scanning system;
**Figures 2a and 2b** show graphs of X-ray energy spectra from different anode materials;
**Figure 3** is a schematic view of a scanning system;
**Figure 4** is a schematic view of an X-ray source forming part of the scanning system of Figure 3;
**Figure 5** is a front view of an anode forming part of the system of Figure 4;
**Figure 6** is a schematic diagram showing operation of the system of Figure 4;
**Figure 7** is a graph of X-ray absorption as a function of energy in a typical filter material;
**Figure 8** is front view of a target according to an embodiment of the invention;
**Figure 9** is graph showing the energy spectrum of X-rays produced from the target of Figure 8;
**Figure 10** is sketch of part of a detector array for use with the target of Figure 8;
**Figure 11** is a sketch of part of a further detector array for use with the target of Figure 8;
**Figure 12** is a graph showing the matching of the filters of Figures 10 and 11 with the target of Figure 8;
**Figure 13** is a front view of a target according to an embodiment of the invention;
**Figure 14** is a front view of a target according to an embodiment of the invention;

### Description of the Preferred Embodiments

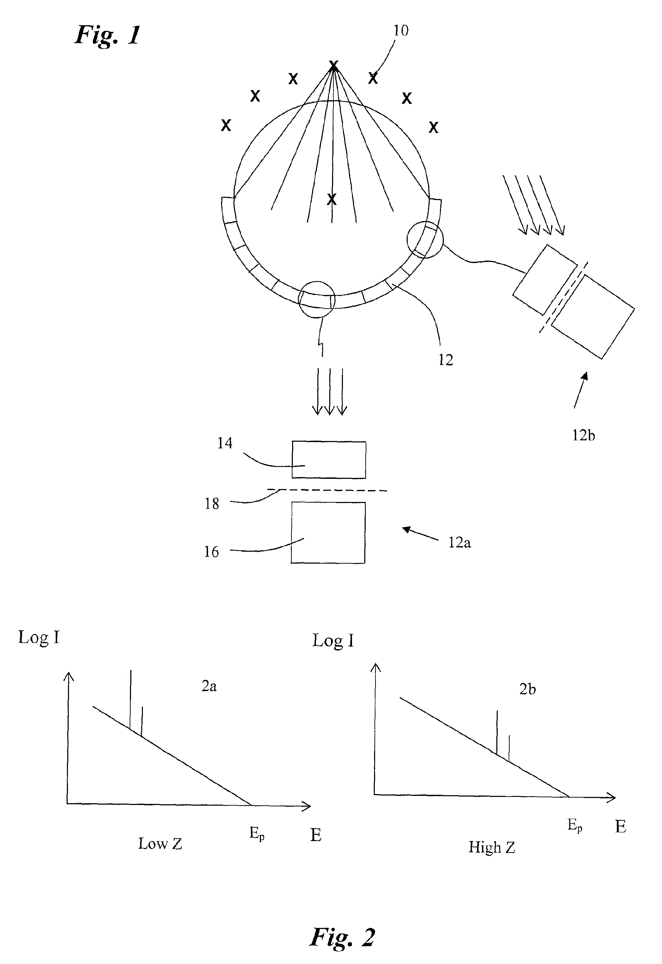

Referring to Figure 1, an X-ray scanner comprises a ring array of X-ray sources 10, offset axially from a ring array of X-ray detectors 12. Each of the sources 10 is activated in turn and, for each source, the signals from the detectors 12 are stored and analysed. Each of the sensors is a known stacked sensor comprising a thin front detector element 14 in front of a thicker rear detector element 16, with a filter 18 between the two detector elements. The front detector element 14 is arranged to detect lower energy X-rays, the rear detector element 16 is arranged to detect higher energy X-rays, and the filter 18 is arranged to filter out the lower energy X-rays not absorbed by the front detector element 14.

Since all of the detectors are aligned with, and face, the axis X of the scanner, it will be seen that at the centre of the X-ray beam, a stacked detector 12a works well in that the front detector element 14, filter 18 and rear detector element 16 are all aligned with the direction of the incident X-ray beam. In contrast, at the edge of the beam in detector 12b, the front detector element 14, filter 18 and rear detector element 16 are not aligned with the beam and there is significant low energy leakage into the rear detector element 16. Similarly, the relatively large material thickness presented to the X-ray beam by the front detector element 14 causes high energy signal leakage into the measured signal. This signal leakage between detector elements compromises the ability of the X-ray system to provide materials discrimination.

Referring to Figure 2, the example provides energy discrimination by using different target materials in the X-ray source thereby modulating the spectral composition of the primary X-ray beam. Figure 2 shows idealised X-ray spectra from an X-ray tube operating at the same tube voltage (with maximum X-ray energy Ep) but in Figure 2a with a low atomic number (Z) anode (such as silver, Ag) and in Figure 2b a high atomic number anode (such as tungsten, W). It is seen that the Bremsstrahlung spectral components are similar in each case, but the characteristic fluorescence lines, which form peaks of high intensity in the energy spectrum, are quite different in energy. The relative position of the fluorescence radiation results in a significantly higher mean spectral energy for the tungsten target than that observed with the silver target. When integrating the transmitted X-ray signal after attenuation by a complex object, the silver anode X-ray beam will be significantly attenuated by high atomic number materials whereas the tungsten spectrum will be less highly attenuated. Taking the ratio of the tungsten to silver anode X-ray data provides an equivalent set of data as is obtained when using two detector sets.

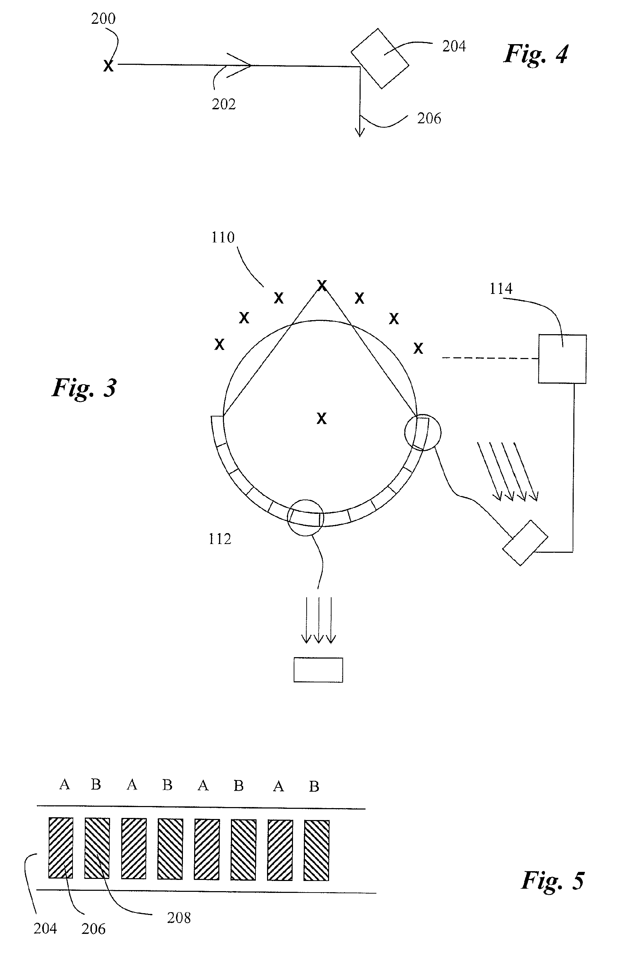

Referring to Figure 3, an X-ray scanner according to an example comprises an array of X-ray sources 110 and an array of X-ray detectors 112 axially offset from the sources. In each case the array is spaced around the axis of the scanner. Each of the sources is arranged to direct beams of electrons towards the axis of the scanner. Each of the detectors is also directed towards the axis of the scanner. In this case each array is a ring array, but either the sources or the detectors may form a partial ring array, or other shaped arrays. However it is desirable that the sources, or the source positions within the sources from which the X-ray beams are generated, are arranged in a common plane perpendicular to the scanner axis. Similarly the detectors are arranged in one or more arrays each in a plane perpendicular to the scanner axis. A control system 114 is arranged to control the sources 110 so that each of them can be activated independently to scan an object in the scanner. Again, the sources 110 are arranged to be activated in turn, but in this case the sources 110 are arranged to generate X-rays with different energy spectra, and the detectors are single element non-stacked detectors. The detectors are all the same, having the same response, i.e. producing the same output in response to X-rays over a range of energies. In this case the detectors are integrating detectors which detect X-rays over a range of energies. They produce an output which varies with the total X-ray intensity over that range. The signal leakage between detectors 112 has been eliminated since the small amount of filtering by adjacent detectors at the edge of the active beam can be taken into account by straightforward calibration factors.

Referring to Figure 4 the X-ray source ring is made up of a number of X-ray tubes, each comprising an electron source 200 arranged to produce a beam 202 of electrons, and an anode 204. The electron source 200 is controllable in known manner to scan the electron beam 202 longitudinally along the anode 204 to generate X-rays 206 from source positions along the anode 204.

Referring to Figure 5, the anode 204 is coated with thin film areas of two different target materials A and B. Here, target material A is patterned onto the anode 204 in rectangular areas or blocks 206, each block being of uniform composition over its own area, in this case being of a single element, and larger than the focal region of the incident electron beam. Interspersed between each target block 206 of material A is a target block 208 which comprises a different target material B. The target material blocks 206, 208 therefore form a linear array of target areas or positions, arranged in a ring around the scanner, with the target material alternating between the two different materials A and B along the array.

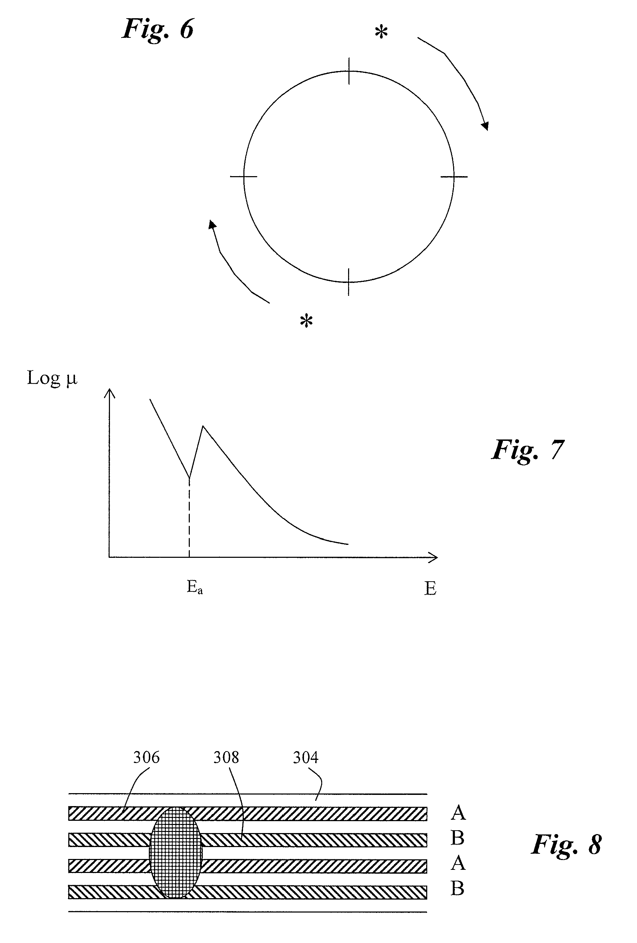

Referring to Figure 6, the scanner can be controlled so that two electron beams are swept around the object under inspection, the two beams being preferably offset by 180 degrees plus the angular displacement between an adjacent pair of A and B type target blocks. The scanning is performed using a switchable electron source which is controlled to switch on an electron beam directed at one of the target areas and then switching it off again, and then switching electron beams directed at each of the other target positions on and off in turn, so that the beam is swept across the target positions in steps. This results in one block of material A and one of material B being targeted simultaneously, the two active target areas being almost opposite each other so that half of the detector array 112 can be used to detect X rays from one of them, and the other half of the detector array 112 to detect X rays simultaneously from the other. Typically, the spacing of the blocks 206 of materials A and B is selected such that the angular sampling rate of both trajectories meets the Nyquist sampling criteria.

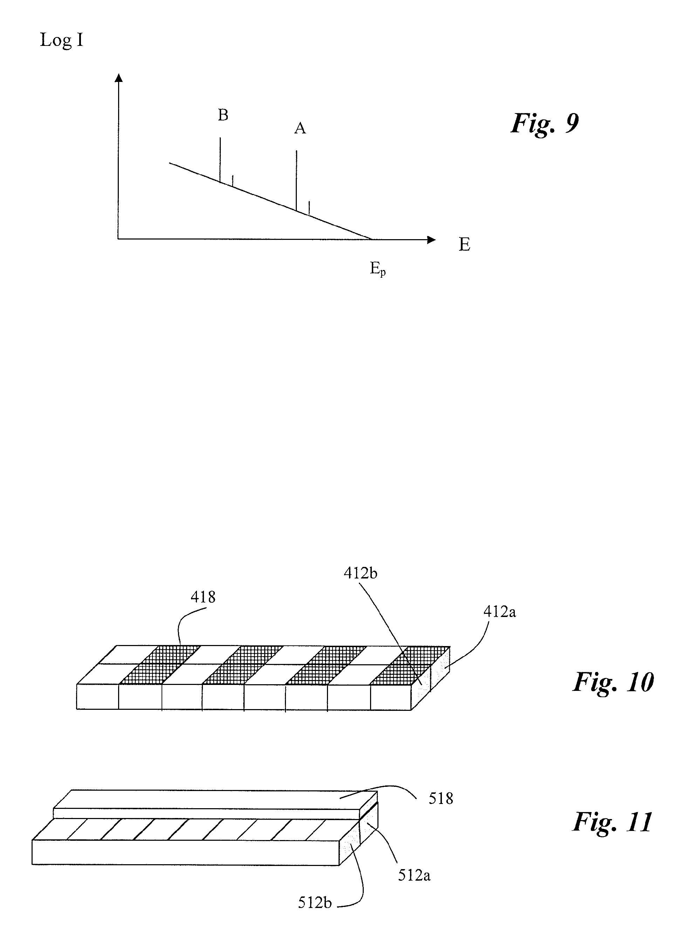

The data from the detectors 112 may then be used to reconstruct independent tomographic images, one for each source type A or B, which can then be subtracted one from the other or otherwise analysed following image reconstruction. Alternatively, the projection data can be combined prior to backprojection to form a modified projection data set. Referring to Figure 7, filters are associated with the detectors so as to vary the response characteristics of the detectors, i.e. the magnitude of their response as a function of X-ray energy, to provide further energy discrimination. To provide discrimination the response characteristics need to of a different shape, i.e. varying differently with energy, not just of a different scale. Figure 7 shows the absorption coefficient µ of a typical filter material as a function of X-ray energy. It can be seen that the absorbing material has an absorption edge at a characteristic energy Ea, this energy being equal to that at which participation of K-shell atomic electrons becomes possible. This results in low absorption at energies just below the characteristic energy Ea and high absorption at energies just above the characteristic energy Ea. This allows the filter material to be used to block some X-ray energies and pass other X-ray energies. By selecting carefully matched target materials with different fluorescent peaks and an appropriate filter material, it is possible to further enhance the selective energy response of the integrating detectors. For example, with suitable filtering, one set of detectors can be made more sensitive than the other to X-rays at the fluorescent peak of one of the target materials, or over a first range of energies which may include that peak, and the other set of detectors can be made more sensitive than the first to X-rays at the fluorescent peak of the other target material, or over a second range of energies which can include that peak. More generally, the ratio of the responses of one of the detector arrays to X-rays at the two fluorescent peak energies is different to the ratio of the responses of the other. Similarly the ratio of the responses of one of the detector arrays to X-rays having the two energy spectra of the two materials is different to the ratio of the responses of the other detector array to X-rays having those energy spectra. As an example, a Tantalum filter strongly absorbs Tungsten characteristic X-rays but is relatively transparent to Molybdenum characteristic X-rays.
Referring to Figure 8, in an embodiment which includes a detector arrangement as in Figure 7, the target area of the anode 304 has parallel strips 306, 308 of target metal A and B formed on it, extending in the direction S in which the electron beam is scanned, and the electron beam is designed to be large enough to irradiate both target metals A and B simultaneously. In this case there are four strips, two of each material A and B, but obviously other numbers of strips can be used. A composite X-ray spectrum is generated having an energy spectrum as that shown in Figure 9 which is the sum of the spectra of the two materials A and B having two peaks each produced by one of the target materials A and B. By placing a suitable filter material at some of the detectors, for example as in Figure 7, it is possible to generate different responses, with different energy dependence, at different detectors.
Referring to Figure 10, a two-ring detector array comprises first and second adjacent rings 412a, 412b of detectors, with individual metal filters 418 placed on every other detector in each ring. Referring to Figure 11, a two ring detector array 512 is used in which a strip of filter material 518 is placed over the whole of one ring 512a and no filter is used on the other ring 512b. Similar patterns may be used on multi-ring detector systems as appropriate. Both of these detector arrangements can be used with the targets of Figure 5 or Figure 8.

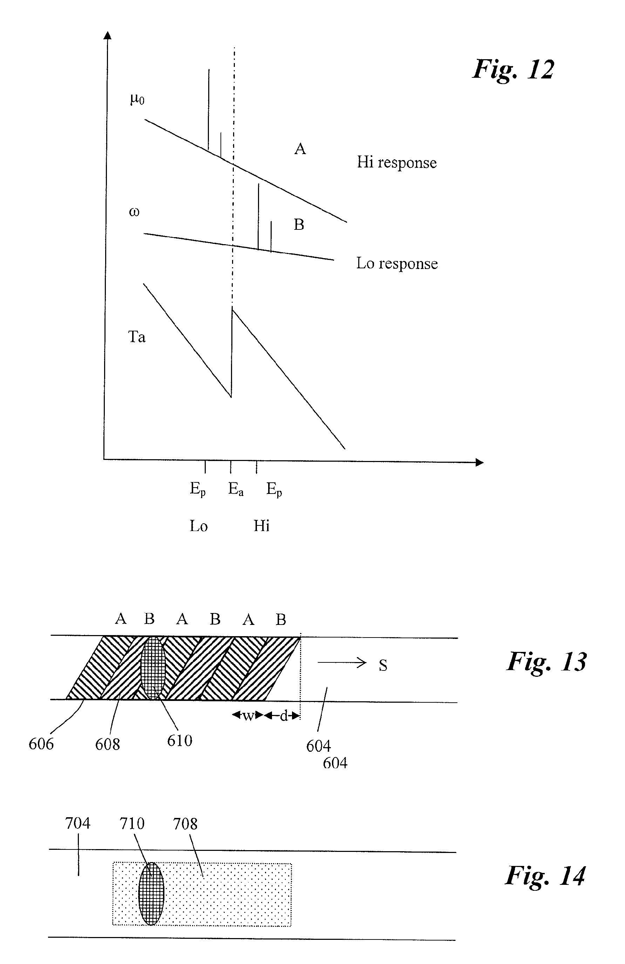

Referring to Figure 12, in either detector arrangement, the filter material is chosen so that absorption edge Ea falls at an energy between the two peaks in the composite X-ray spectrum. As described above, if Mo and W are used as the two target materials, then Ta can be used as a suitable filter material.

It will be appreciated that the combination of two target materials with different X-ray energy spectra having different peaks, and a suitably selected filter, provides quasi mono-energetic imaging for improved accuracy materials analysis, as it provides a high degree of differentiation between the outputs from the two sets of detectors, which is highly dependent on the absorption spectrum of the object being scanned, and therefore provides a good degree of, for example, discrimination between objects of different materials.

Complex targets can be made through the use of sputter coating. Using multiple sputter targets and shadow or other masking technologies, it is straightforward to pattern any of the target configurations. To ensure good adhesion between the target metal A, B and the segmented anode base metal, it is possible to either alloy or diffuse the target metal into the anode base. Typically this alloying or diffusion process is conducted in a vacuum or hydrogen filled furnace operating at suitably high temperatures (typically in the range 500 - 1000C). Depending on the process conditions chosen, a thin interface layer can be formed or the entire target metal can be alloyed into the base metal.

As a further refinement of this process, a multi-layer target can be formed using, for example, sputter coating of thin films (10 - 100 angstrom for example) of various different coating metals (tungsten and uranium, for example) one on top of another. The multi-layer target can then be alloyed or diffused together and into the base metal in order to form a target that produces reasonably complex X-ray spectra during electron bombardment.

Referring to Figure 13, in a further embodiment the two target materials A and B of different atomic number Z are again arranged in alternate areas or strips 606, 608 along the anode 604, but in this case the strips of target material are arranged at an oblique angle, in this case about 45°, to the direction of scanning S of the electron beam. The strips 604, 608 are narrow enough, and angled such that the offset d between their upper and lower ends, in the direction S of scanning of the electron beam, is at least equal to their width w. This means that the electron beam 610, that is large enough to extend across substantially the full width of the target area, and as wide in the scanning direction S as the strips, will always cover an area of the target that is made up of approximately equal areas of the two target materials. This allows a detector array similar to that of Figure 10 or Figure 11 to be used.

Referring to Figure 14, in a further embodiment the target area of the anode 704 is covered with a mixture 708 of two target materials A and B with different atomic number. This again means that when the electron beam 710 strikes the target, the X-ray beam generated has as spectrum with two peaks in it, which can be separated by filtering for detection, for example using the detector arrangements of Figure 11 or Figure 12.

It will be appreciated that, in embodiments described in which two target materials are used, it would be possible in some circumstances to use three or more target materials to obtain further energy discrimination.

## Claims

1. An X-ray scanner comprising an electron source and an anode, the anode (204; 304; 604; 704) having a target surface formed from a plurality of different materials (206, 208; 306, 308; 606, 608; 708), the electron source being arranged to direct a beam of electrons at the target surface so as to generate, simultaneously, X-rays of two different energy spectra from two of the different materials, and two detector arrays (12) having different energy response characteristics.

2. A scanner according to claim 1 wherein the ratio of the responses of one of the detector arrays to X-rays having the two energy spectra of the two materials is different to the ratio of the responses of the other detector array to X-rays having those energy spectra.

3. A scanner according to claim 1 or claim 2 wherein the detectors of one of the arrays of detectors include a filter material and a detector element.

4. A scanner according to claim 3 wherein the two materials are each arranged to generate X-rays having intensity peaks at a respective fluorescent energy, and the filter material is arranged to provide different attenuations at the two fluorescent energies.

5. A scanner according to claim 4 wherein the filter material has an absorption edge at a frequency between the two fluorescent energies.

6. A scanner according to any of claims 3 to 5 wherein the detectors of both the detector arrays include identical detector elements

7. A scanner according to any of claims 1 to 6 wherein the target surface has a plurality of material areas each formed from one of the materials, and the electron source is arranged to direct a beam of electrons at a target area of the target, the target area comprising part of at least two of the material areas.

8. A scanner according to claim 7 wherein the electron source is arranged to direct electrons at a plurality of target areas spaced along the target.

9. A scanner according to claim 8 wherein the material areas are arranged as parallel strips each extending through a plurality of the target areas.

10. A scanner according to any of claims 1 to 6 wherein the target surface is formed from a mixture of the materials.

## Patentansprüche

1. Röntgenscanner, eine Elektronenquelle und eine Anode umfassend, wobei die Anode (204; 304; 604; 704) eine Zieloberfläche aufweist, die aus einer Mehrzahl verschiedener Materialien (206, 208; 306, 308; 606, 608; 708) gebildet ist, wobei die Elektronenquelle so angeordnet ist, dass sie einen Strahl aus Elektronen auf die Zieloberfläche lenkt, um gleichzeitig Röntgenstrahlen mit zwei verschiedenen Energiespektren aus zwei von den verschiedenen Materialien zu bilden, und zwei Detektorgruppen (12), die verschiedene Energieantwortcharakteristika aufweisen.

2. Scanner nach Anspruch 1, wobei das Verhältnis der Antworten von einer der Detektorgruppen auf Röntgenstrahlen, welche die zwei Energiespektren der zwei Materialien aufweisen, verschieden ist von dem Verhältnis der Antworten der anderen Detektorgruppe auf Röntgenstrahlen mit diesen Energiespektren.

3. Scanner nach Anspruch 1 oder Anspruch 2, wobei die Detektoren von einer der Gruppen von Detektoren ein Filtermaterial und ein Detektorelement beinhalten.

4. Scanner nach Anspruch 3, wobei die beiden Materialien jeweils so angeordnet sind, dass sie Röntgenstrahlen mit Intensitäts-Peaks bei einer entsprechenden Fluoreszenzenergie erzeugen, und das Filtermaterial so angeordnet ist, dass es bei den zwei Fluoreszenzenergien unterschiedliche Dämpfungen bereitstellt.

5. Scanner nach Anspruch 4, wobei das Filtermaterial eine Absorptionsgrenze bei einer Frequenz zwischen den beiden Fluoreszenzenergien aufweist.

6. Scanner nach einem der Ansprüche 3 bis 5, wobei die Detektoren beider Detektorgruppen identische Detektorelemente aufweisen.

7. Scanner nach einem der Ansprüche 1 bis 6, wobei die Zieloberfläche eine Mehrzahl von Materialbereichen aufweist, die jeweils aus einem von den Materialen gebildet sind, und die Elektronenquelle so angeordnet ist, dass sie einen Strahl aus Elektronen auf einen Zielbereich des Ziels lenkt, wobei der Zielbereich einen Teil von mindestens zwei von den Materialbereichen umfasst.

8. Scanner nach Anspruch 7, wobei die Elektronenquelle so angeordnet ist, dass sie Elektronen auf eine Mehrzahl von Zielbereichen lenkt, die entlang des Ziels beabstandet sind.

9. Scanner nach Anspruch 8, wobei die Materialbereiche als parallele Streifen angeordnet sind, die jeweils durch eine Mehrzahl der Zielbereiche hindurch verlaufen.

10. Scanner nach einem der Ansprüche 1 bis 6, wobei die Zieloberfläche aus einer Mischung der Materialien gebildet ist.

## Revendications

1. Un scanner à rayons x comprenant une source d'électrons et une anode, l'anode (204, 304, 604, 704) possédant une surface cible formée à partir d'une pluralité de matériaux différents (206, 208, 306, 308, 606, 608, 708), la source d'électrons étant agencée de façon à diriger un faisceau d'électrons au niveau de la surface cible de façon à générer, simultanément, des rayons x de deux spectres d'énergie différents provenant de deux des matériaux différents, et deux matrices de détecteurs (12) possédant des caractéristiques de réponse d'énergie différentes.

2. Un scanner selon la Revendication 1 où le rapport des réponses de l'une des matrices de détecteurs sur des rayons x possédant les deux spectres d'énergie des deux matériaux est différent du rapport des réponses de l'autre matrice de détecteurs sur des rayons x possédant ces spectres d'énergie.

3. Un scanner selon la Revendication 1 ou 2 où les détecteurs de l'une des matrices de détecteurs comprennent un matériau de filtre et un élément détecteur.

4. Un scanner selon la Revendication 3 où les deux matériaux sont chacun agencés de façon à générer des rayons x possédant des pics d'intensité à une énergie fluorescente respective, et le matériau de filtre est agencé de façon à fournir des atténuations différentes aux deux énergies fluorescentes.

5. Un scanner selon la Revendication 4 où le matériau de filtre possède une discontinuité d'absorption à une fréquence entre les deux énergies fluorescentes.

6. Un scanner selon l'une quelconque des Revendications 3 à 5 où les détecteurs des deux matrices de détecteurs comprennent des éléments détecteurs identiques.

7. Un scanner selon l'une quelconque des Revendications 1 à 6 où la surface cible possède une pluralité de zones de matériau, chacune d'elles étant formée à partir de l'un des matériaux, et la source d'électrons est agencée de façon à diriger un faisceau d'électrons vers une zone cible de la cible, la zone cible comprenant une partie d'au moins deux des zones de matériau.

8. Un scanner selon la Revendication 7 où la source d'électrons est agencée de façon à diriger des électrons vers une pluralité de zone cibles espacées le long de la cible.

9. Un scanner selon la Revendication 8 où les zones de matériau sont agencées sous la forme de bandes parallèles, chacune d'elles s'étendant au travers d'une pluralité des zones cibles.

10. Un scanner selon l'une quelconque des Revendications 1 à 6 où la surface cible est formée à partir d'un mélange des matériaux.
